# EUROPEAN PATENT APPLICATION

(11) **EP 3 354 156 A1**
(43) Date of publication of application: **01.08.2018**
(21) Application number: 16849029.0
(22) Date of filing: 23.09.2016
(51) Int. Cl.: A45D 44/00, A45D 44/22, A61K 8/02, A61M 5/178, B65D 81/32, B65D 75/58, B65D 75/28

(54) **FACIAL MASK KIT**

(30) Priority: 25.09.2015 KR 20150136926
(71) Applicant: Oozoo Skin Science Co., Ltd., Seoul 07236 (KR)
(72) Inventor: LEE, Ha Jun, Seoul 07064 (KR)
(74) Representative: Potter Clarkson LLP
(86) International application number: PCT/KR2016/010712
(87) International publication number: WO 2017/052304

(57) **Abstract**

The objective of the present invention to provide a facial mask kit in which an appropriate amount of a skin care agent is uniformly distributed on a facial mask sheet, thereby increasing the convenience of use of the facial mask and reducing the waste of the skin care agent. Another objective of the present invention is to provide a facial mask kit capable of maximizing the efficacy of each component of the skin care agent by distributing the skin care agent comprising a plurality of components on the facial mask sheet immediately before use.

## Description

### Technical Field

The present invention relates to a facial mask kit and, more particularly, a facial mask kit that can stably keep a skincare agent absorbed in facial mask sheets for beauty and provide a maximum skincare function when the skincare agent is used.

Facial mask sheets for skin beauty etc. are formed by soaking sheets designed and cut to be fitted to faces with a skincare agent and are usually packed in a pouch for sale on the market after being soaked with a skin care agent. Accordingly, users use the facial masks by opening a pouch, taking out a facial mask soaked with a skincare agent, and the putting the facial mask on their faces.

However, there are the following problems when using facial masks.

First, an excessive amount of skincare agent is applied to a sheet in a process of sufficiently soaking the sheet with the skincare agent when a product is manufactured, so when a user opens a pouch, the skincare agent flows down from the sheet, thereby causing inconvenience.

Second, facial masks are generally used for various purposes such as supplying nutrients, supplementing moisture, and stabilizing skin, and the components of skincare agents are changed in accordance with the purposes. However, when several components are mixed and time passes, the components generate a reaction, so the effective components may be disrupted or deteriorated.

### Background Art

There is Korean Patent No. 1176723 ("Cosmetic pack", 2012.08.17, hereafter, referred to as 'prior art document) for solving the first one of the problems in the related art. According to the prior art document, a space of a facial mask pouch is divided into two sections by a breakable membrane so that a sheet is kept in one of the sections and a skincare agent is kept in the other section. Further, when a user presses the space keeping the skincare agent, the breakable membrane is ruptured and the sheet is soaked with the skincare agent. Accordingly, the amount of skincare agent to soak the sheet can be adjusted in accordance with the pressing degree by the user. However, according to the prior art document, after the breakable membrane between the skincare agent space and the sheet space is ruptured, the skincare agent flows out of the skincare agent space without stopping, so even though the user does not presses the skincare agent space, the skincare agent keeps naturally flowing out, and accordingly, the sheet is soaked with an excessive amount of skincare agent. Further, according to the prior art document does not consider a measure for solving the second problem.

Further, as for the second problem, that is, the problem that when a plurality of skincare agents are used, the effects are deteriorated due to reaction of the components of the skincare agents, so currently, all manufacturers attempt to solve the problem by separately manufacturing products for each component. Accordingly, customers have to purchase several products and separately used the products, depending on the desired skincare functions, so economical and time waste is increased.

### Documents of Related Art

[Patent Document]

1. Korean Patent No. 1176723 ("Cosmetic pack", 2012.08.17)

### Disclosure

### Technical Problem

Accordingly, the present invention has been made in an effort to solve the problems of the related art and an object of the present invention is to provide a facial mask kit that can increase convenience in use of a facial mask and prevent waste of a skincare agent by uniformly soaking a facial mask sheet with an appropriate amount of skincare agent. Another object of the present invention is to provide a facial mask kit that can maximize the effects of the components of a plurality of skincare agents by soaking a sheet with the skincare agents immediately before using sheet.

### Technical Solution

In order to achieve the object, a facial mask kit 1000 includes: a pouch 100 having a sheet space 110 for keeping a sheet 400 therein; an injector 200 including a cylinder having an outlet at a first side and keeping a first skincare agent therein and a piston inserted in the cylinder from a second side of the cylinder; an injection port 300 disposed on a first side of the pouch 100 to connect inside and outside of the sheet space 110, in which the skincare agent is injected into the sheet space 110 through the injection port 300 by the injector 200 to uniformly soak the sheet 400.

The injector 200 may include a first cylinder 210, a second cylinder 220 inserted in the first cylinder 210, and a piston 230 inserted in the second cylinder 220, a first skincare agent may be kept in a space between the first cylinder 210 and the second cylinder 220, and a second skincare agent may be kept in a space between the second cylinder 220 and the piston 230.

The first cylinder 210 may have: a first body 211 having a first space 211s therein and having a first side and a second side communicating with each other; an outlet 213 formed at a first end of the first body 211; a first flange 212 formed at a second end of the first body 211; and a first locking portion 215 protruding in a ring shape from an inner side of the first body 211. The first locking portion 215 may be formed in a pair of ring shapes protruding from the inner side of the first body 211 and positioned close to each other.

The second cylinder may have: a second body 221 having a second space 221s therein, having a first side and a second side communicating each other, and inserted in the first body 211; an outlet 223 formed at a first end of the second body 221 and having a larger diameter at an end than a diameter of the second body 221; a second flange 222 formed at a second end of the second body 221; a breakable membrane 224 formed like a separation membrane closing the outlet 223; and at least one second locking portion 225 protruding in a ring shape from an inner side of the second body 221. The second locking portion 225 may be formed in a pair of ring shapes protruding from the inner side of the second body 221 and positioned close to each other.

The piston 230 may have: a piston body 231 inserted in the second body 221; a breaking portion 233 formed at a first end of the piston body 231 and pointed at an end thereof; a piston flange 232 formed at a second end of the piston body 231; and at least one piston locking portion 235 protruding in a ring shape from an outer side of the piston body 231. The piston body 231 may have a space 231s connecting a first side and second side of the piston body 231 and may have a closing wall 234 formed like a separation wall to close the space 231s at a position closer to the first side of the piston body 231.

The injection port 300 may have: an injection port body 310 having an empty space therein and having a first side and a second side communicating with other; an injection port flange 320 formed at a first end of the injection port body 310 and attached and fixed to the first side of the pouch 110; a muzzle 315 formed at a second end of the injection port body 310; and a cap 330 disposed over the muzzle 315 to be opened and closed.

The injection port body 310 may be inclined with respect to a surface of the pouch 110. In the facial mask kit 1000, the sheet 400 may be positioned closer to the bottom in the sheet space 110 and the injection port 300 may be positioned closer to the top in the sheet space 110. The injection port body 310 may be declined downward.

The pouch 100 may further have an injector space 120 separated from the sheet space 110. The pouch 100 may be formed by stacking a pair of packaging materials and then thermally bonding edges to form the sheet space 110. The injector space 120 separated from the sheet space 110 of the pouch 100 may be formed by further thermally bonding centers of the pair of packaging materials with the edges thermally bonded.

A perforated crease may be formed at the bonded portion 130 between the sheet space 110 and the injector space 120.

A pair of notched opening portions 140 may be formed at left and right ends of an upper portion of the pouch 100.

In the pouch 100, a packaging material for the sheet space 110 is opaque, and a packaging material for the injector space 120 is transparent at least on one side.

The injector 200 may have an injector cap 240 sealing the outlet. The injector cap 240 may be thread-fastened to the outlet. The injector cap 240 may have wings to be turned when being thread-fastened to the outlet.

### Advantageous Effects

According to the present invention, there is provided a facial mask that is soaked with a skincare agent and attached to the face to improve the status of the skin. A pouch keeping a sheet and an injector keeping a skincare agent are separately provided, so a user can appropriately adjust the amount of a skincare agent to be applied to a sheet as he/she wants. Accordingly, it is possible to eliminate inconvenience for a user due to the phenomenon that a sheet is soaked with an excessive amount skincare agent and the skincare agent flows down from the sheet. Further, according to the present invention, when a skincare agent is injected, the skincare agent spreads around the center portion of a sheet, so the skincare agent is uniformly distributed in the sheet. Accordingly, it is possible to effectively prevent a skincare agent from flowing down and apply the function of the skincare agent uniformly to the entire skin. In addition, it is possible to prevent an excessive amount of skincare agent from being provided for one facial mask, so manufactures can reduce the manufacturing cost and customers can obtain an economical effect through a reduced product price.

Further, according to the present invention, there is a remarkable effect that it is possible to use a plurality of skincare agents having different components. In detail, in the related art, when skincare agents are mixed, the components are mixed and the functions are deteriorated or the effective components are disrupted or deteriorated by reaction of the components. However, according to the present invention, since two or more components are separately kept in the injector, it is possible to prevent mixing and deterioration of the components when keeping them. Further, a sheet is soaked with a plurality of skincare agents having different components immediately before being used, so the components can be applied to the skin before reaction of the components occurs, and accordingly, it is possible to maximize the effects of the skincare agents. In addition, according to the present invention, since it is possible to simultaneously use a plurality of components, a user can reduce economical and time waste that is caused by separately purchasing a plurality of facial masks and use them for a long time. Manufacturers also can change the contents of a product by changing the components to be kept in the injector, so no separate manufacturing line for each component is required and the cost for facilities can be remarkably reduced.

### Description of Drawings

FIG. 1 shows an example of a facial mask kit of the present invention.
FIG. 2 shows an embodiment of the detailed configuration of an injection port of the facial mask kit of the present invention
FIGS. 3 to 7 show an embodiment of the detailed configuration of an injector of the facial mask kit of the present invention.
FIG. 8 shows various embodiments of injecting a skincare agent using the injector.

### ** Reference Numerals **

1000: Facial mask kit (of invention)
100: Pouch
110: Sheet space 120: Injector space
130: Bonded portion 140: Opening portion
200: Injector
210: First cylinder
211: First body 211s: First space
212: First flange 213: Outlet
214: Coupling portion 215: First locking portion
220: Second cylinder
221: Second body 221s: Second space
222: Second flange 223: Outlet
224: Breakable membrane 225: Second locking portion
230: Piston
231: Piston body 231s: Space
232: Piston flange 233: breaking portion
234: Closing wall 235: Piston locking portion
240: Injector cap
300: Injection port
310: Injection port body 315: Muzzle
320: Injection port flange 330: Cap
400: Sheet

### Mode for Invention

Hereinafter, a facial mask kit having the configuration described above in accordance with the present invention is described in detail with reference to the accompanying drawings.

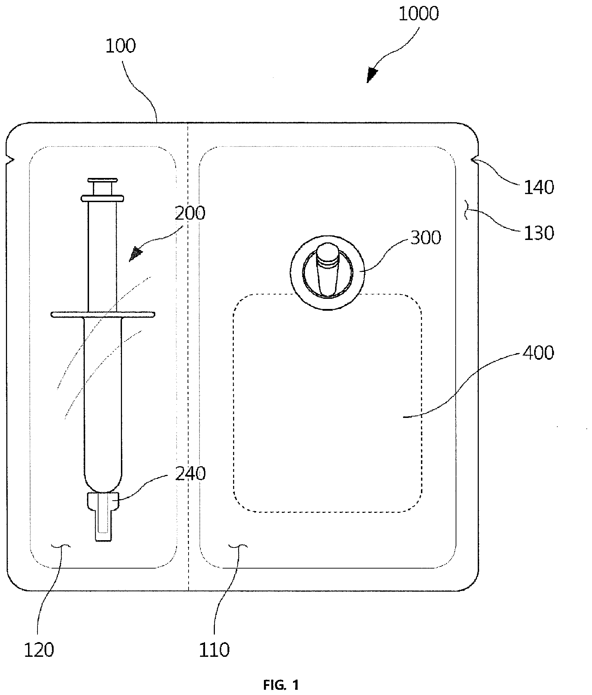

FIG. 1 is an embodiment of a facial mask kit of the present invention and shows the entire configuration of a facial mask kit 1000 of the present invention. Referring to FIG. 1, the facial mask kit 1000 of the present invention fundamentally includes a pouch 100, an injector 200, and an injection port 300. The components are described in detail hereafter.

A sheet space 110 is formed in the pouch 100 and a sheet 400 is stored in the sheet space 110. As described above, an object of the present invention is to solve the problem in the related art that an excessive amount of skincare agent is absorbed in a sheet, thereby causing inconvenience, and to enable a user to soak a sheet with an appropriate amount of skincare agent, as he/she wants. Accordingly, the sheet 400 is kept without a skincare agent absorbed therein in the sheet space 110.

The injector 200, which is similar to a syringe carrying a skincare agent therein, includes a cylinder having an outlet at a first side and a piston inserted in a second side of the cylinder. Similar to a common syringe, when a user pushes the piston into the cylinder, the skincare agent in the cylinder is discharged through the outlet. The detailed shape of the injector 200 will be described in detail below.

The injection port 300 is formed on a first surface of the pouch 1 and connects the inside and outside of the sheet space 110 to each other. A cap that can cover/open the injection port 300 is provided for the injection port 300. The cap is closed at normal times and a user opens the cap when injecting a skincare agent, using the injector 200.

According to the facial mask sheet 1000 having this configuration of the present invention, a skincare agent is injected into the sheet space 110 through the injection port 300 by the injector 200 and permeated into the sheet 400, whereby the sheet is soaked with the skincare agent. That is, the sheet 400 is in a dried state before a skincare agent is injected by the injector 300 and a user can inject a desired appropriate amount of skincare agent, using the syringe-shaped injector 200. Accordingly, it is possible to completely remove the problem that a sheet is soaked with an excessive amount of skincare agent and the skincare agent flows down in use, thereby causing inconvenience.

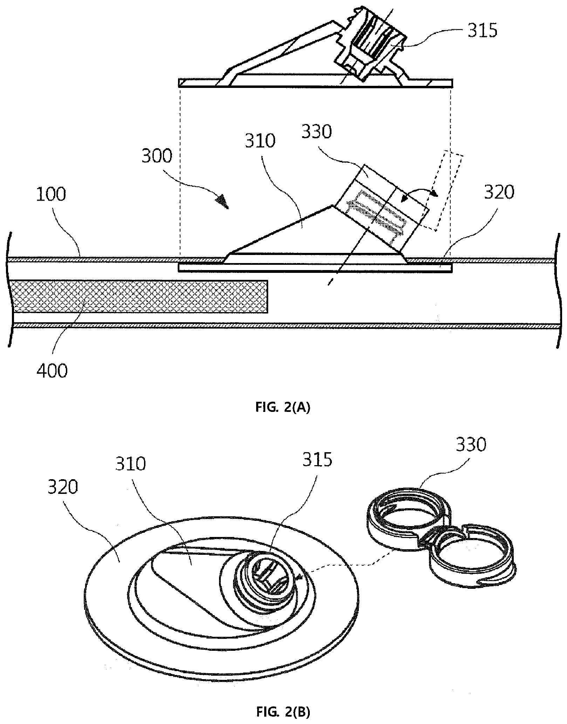

The detailed configuration of the pouch 100 and the injection port 300 for smoothly and uniformly distributing a skincare agent into sheet 300 is described in detail with reference to FIGS. 1 and 2.

FIG. 2 shows an embodiment of the detailed configuration of the injection port of the facial mask kit of the present invention. The injection port 300 may include: an injection port body 310 having an empty space therein and having first and second sides communicating with each other; an injection port flange 320 formed at a first end of the injection port body 310 to be attached and fixed to the first surface of the pouch 110; a muzzle 315 formed at a second end of the injection port body 310; and a cap 330 disposed over the muzzle 315 to be opened and closed. The injection port body 310, as shown in FIG. 2(A), may be formed at an angle with respect to the surface of the pouch 110. When liquid is injected into a space, the fluid sensibly vertically flows down due to gravity. However, since the sheet space 110 is formed flat, when a skincare agent is injected perpendicular to the surface of the pouch 100, it is difficult to smoothly inject the fluid and the flow direction is also difficult to control. Further, since the pouch 100 is formed very flat, it is difficult to design and attach the injection port structure in order to inject a skincare agent in parallel with the surface of the pouch 100. However, in the present invention, the injection port 300 is attached to the first surface of the pouch 110 and the injection port body 310 is inclined, as shown in FIG. 2 so that when a skincare agent is injected by the injector 200, the skincare agent flows at an angle by the shape of the injection port body 310. Accordingly, the skincare agent can be smoothly and naturally injected.

Further, according to the facial mask kit 1000, as shown in FIG. 1, the sheet 400 may be positioned closer to the bottom in the sheet space 110 and the injection port 300 may be positioned closer to the top in the sheet space 110. Further, the injection port body 310 may be declined downward. Accordingly, a skincare agent injected through the injection port 300 naturally flows down, so the entire sheet 400 can be uniformly soaked with the skincare agent.

According to the configuration of the present invention, it is possible to separately keep a sheet and a skincare agent and a user can smoothly and uniformly soak the sheet with the skincare agent by adjusting the amount of the skincare to be injected at a desired level. Therefore, it is possible to remove one of the problems in the related art that a sheet is excessively soaked with a skincare agent and the skincare agent flows down in use.

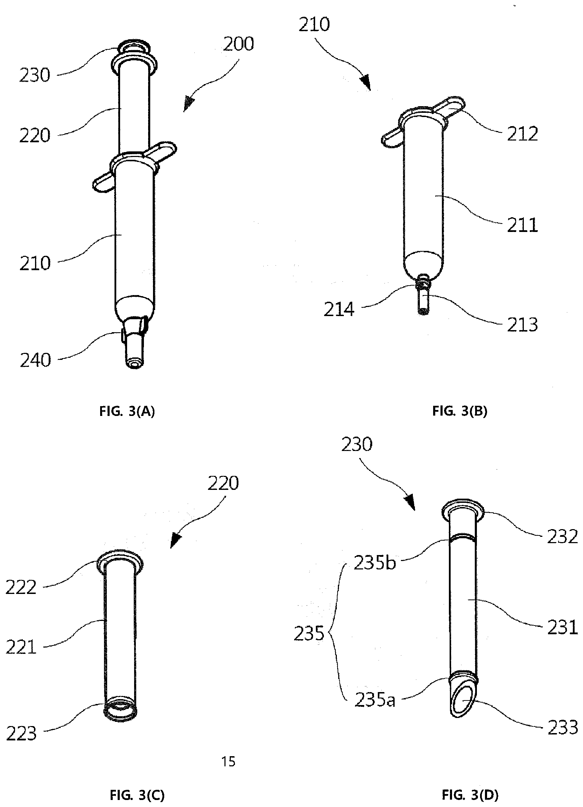

An embodiment of the inject of the present invention for solve the other problems in the related art, that is, the problem that it is impossible to a plurality of skincare agents having several functions is described hereafter with reference to FIGS. 3 to 7.

The injector 200 was described as being formed similar to a syringe. Common syringes have one space therein, so they can keep only one kind of skincare agent. An injector 200 according to an embodiment of the present invention shown in FIGS. 3 to 7 is formed like a double syringe, so it can have two independent spaces. Accordingly, it is possible to keep different components of skincare agents in the independent spaces and the skincare agents can be mixed immediately before being used to soak a sheet. As described above, the reason that it was impossible to use skincare agents having several functions is because if time passes after several components are mixed, they react with each other, so efficient components are disrupted or deteriorated, and accordingly, it is difficult to obtain desired effects in use. However, according to the present invention, a plurality of components of skincare agents are safely kept without contact in independent spaces, so it is possible to completely prevent efficient components from being disrupted or deteriorated due to mixing. Further, the skincare components are mixed immediately before they are used, so they can be applied to the skin before effective components are deteriorated by reacting with each other. Consequently, it is possible to effectively apply the effects of a plurality of skincare agent to the skin.

The configuration of the injector 200 is described in more detail.

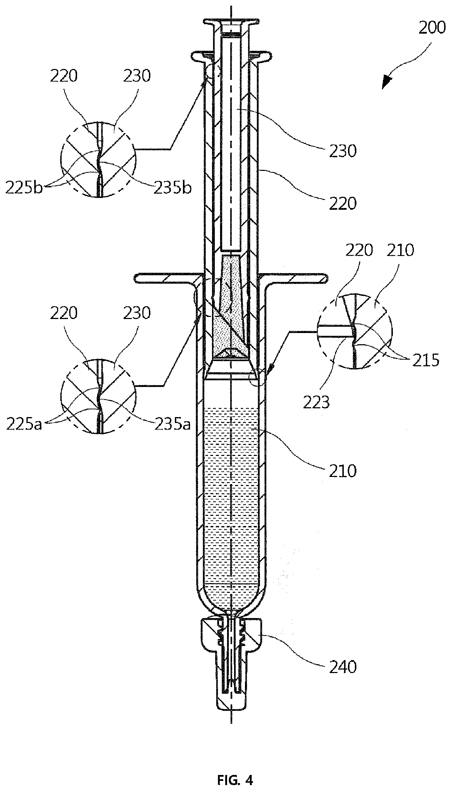

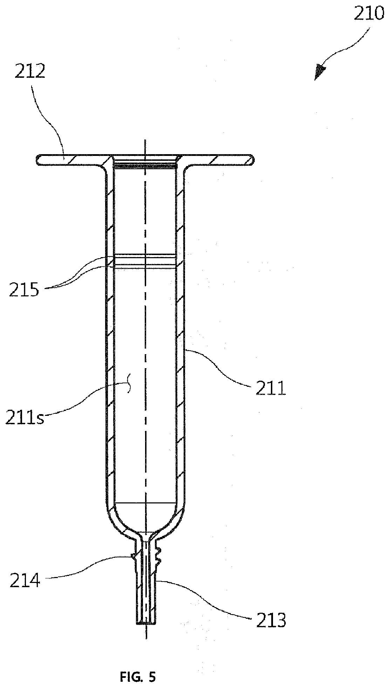

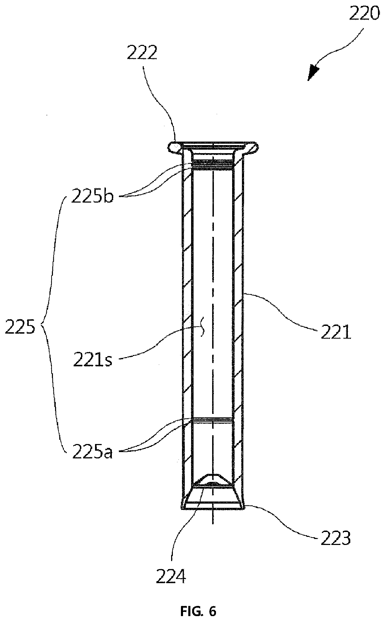

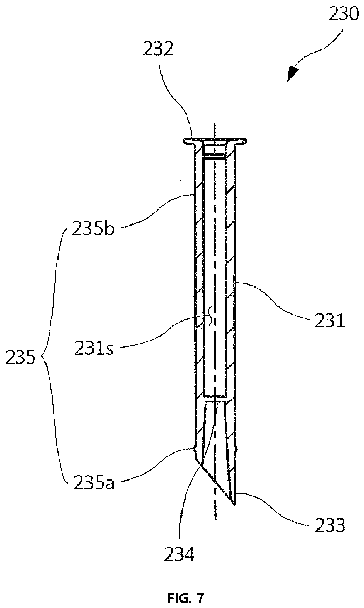

The injector 200, as shown in FIGS. 3(A) and 4, a first cylinder 210, a second cylinder 220 inserted in the first cylinder 210, and a piston 230 inserted in the second cylinder 22. A first skincare agent is kept in the space between the first cylinder 210 and the second cylinder 220 and a second skincare agent is kept in the space between the second cylinder 220 and the piston 230. FIG. 3(A) is a perspective view of the assembly of the injector 200 with all components thereof combined, FIG. 3(B) is a perspective view of the first cylinder 210, FIG. 3(C) is a perspective view of the second cylinder 220, and FIG. 3C is a perspective view of the piston 230. Further, FIG. is a cross-sectional view of the assembly of the injector 200 with all components thereof combined, FIG. 5 is a cross-sectional view of the first cylinder 210, FIG. 6 is a cross-sectional view of the second cylinder 220, and FIG. 7 is a cross-sectional view of the piston 230. That is, the injector 200 of the embodiment shown in FIGS. 3 to 7 is a double syringe. The components and coupling relationships of the components are described in detail.

First, the first cylinder 210 is described. As show in FIG. 5, the first cylinder 210 has a first body 211, a first flange 212, an outlet 213, and a first locking portion 215.

The first body 211 is formed in a tube shape that can keep liquid, similar to the cylinder of a common syringe, that is, the first body 211 has a first space 211s therein and has first and second sides communicating each other. When the injector 200 is assembled by combining the components, a first skincare agent is kept in the first space 211s.

The outlet 213 is formed at a first end of the first body 211 and the flange 212 is formed at a second end of the first body 211. Similar to a common syringe, the outlet 213 is formed with a diameter very smaller than that of the first body 211 so that fluid is not easily discharged unless pressure is applied to the first space 211s. Further, similar to a common syringe, the first flange 212 functions as a support that supports the hand of a user pressing the first cylinder 210.

As described above, liquid is not easily discharged through the outlet 213 unless pressure is applied to the first space 211s, but if the injector is stored for a long period of time with the outlet 213 open, a very small amount of air comes in contact with the first skincare agent in the first space 211s, so there is a possibility of deterioration or disruption of the first skincare agent. Further, there is a possibility that pressure is instantaneously applied to the first space by unexpected external force and the liquid is discharged through the outlet 213. In order to prevent theses problems, as shown in Fig. 1, the injector 200 further includes an injector cap 240 for covering the outlet 213.

The injector cap 230 may be thread-fastened to the outlet 213, as shown in Fig. 4, to stably, firmly, and easily close the outlet. In this case, a coupling portion 214 for thread-fastening is formed on the outer side of the outlet 213, as shown in FIG. 5. Further, in this case, the injector cap 240 has to be turned to be coupled to the outlet 213, so wings may be formed on the injector cap 240, as shown in FIGS. 1, 3, and 4, to easily turn the injector cap.

The part that pressurizes the first space 211s to discharge the first skincare agent is, as shown in FIGS. 3 and 4, the second cylinder 220 inserted in the first body 211. The second cylinder 220 is fitted in close contact with the inner side of the first body 211, similar to the piston of a common syringe, so it is not moved unless specific pressure is applied. However, unlike a common syringe, the first skincare agent should be kept for a long period of time in the first body 211 (that is, in the first space 211s), so there is a possibility that the first space 211s is unexpectedly pressurized by unexpected external shock, so there is a need for a measure against this problem.

The first locking portion 215 is provided as the measure. The first locking portion 215 is formed in a ring shape protruding from the inner side of the first body 211 and fixes the position of the second cylinder 220 so that the second cylinder 215 is not inserted deeper. Obviously, if pressure over a critical level is applied, the second cylinder 220 may be inserted deeper beyond the first locking portion 215 and the first skincare agent in the first space 211s may be naturally discharged through the outlet 213. However, when pressure under the critical level is applied, the second cylinder 220 is locked to the first locking portion 215 and fixed at the position, so it is possible to effectively prevent the first skincare agent from being unnecessarily discharged.

The locking portion 215 is provided, as described above, to fix the position of the second cylinder 220 so that the second cylinder 220 prevented from being inserted deeper than a predetermined position and unnecessarily discharging the skincare agent, so the locking portion 215 may be formed in one ring shape at the predetermined position where the second cylinder 220 has to be stopped. However, if the second cylinder 220 is moved beyond the predetermined position, a problem that the pressure in the first space 211s drops and air flows through the outlet 213 may occur. In order to solve the problem the first locking portion 215 may be formed in a pair of ring shapes protruding from the inner side of the first body 211 and positioned close to each other, as shown in FIG. 4. In this case, the end of the second cylinder 220 is locked and fixed between the pair of rings.

Next, the second cylinder 220 is described. As show in FIG. 5, the second cylinder 220 has a second body 221, a second flange 222, an outlet 223, a breakable membrane 224, and a second locking portion 225.

The second body 221, similar to the first body 211 of the first cylinder 210, has a second space 221s therein and has first and second sides communicating each other. The second body 221 is inserted in the first body 211 to function as a piston of the first cylinder 210.

The second flange 222 is formed at the second end of the second body 221, and similar to the first flange 212, functions as a support that supports the hand of a user pressing the second cylinder 220. The second flange 222 can also limit the insertion depth of the second cylinder 220 by being locked to the first flange 212 when the second cylinder 220 is inserted at a predetermined depth in the first cylinder 210.

A second skincare agent is kept in the second space 221s. As described above, the second skincare agent can be discharged when the first and second sides of the second space 221s are opened, so the parts of the second cylinder 220 should be configured to prevent leakage of the second skincare agent. Since the piston 230 is inserted in the second side of the second cylinder 220, as shown in FIGS. 3 and 4, leakage of the second skincare agent can be prevented. However, the second skincare agent can be discharged from the first side of the second cylinder 220 when a user wants the use it, and the second skincare agent in the second space 221s and the first skincare agent in the 211s should be able to be separated without mixing at normal times. The shape of the outlet 223 and the breakable membrane 224 are configured to ensure this function.

The outlet 223 is formed at the first end of the second body 221 and has a diameter larger than that of the second body 221. Accordingly, the second body 221 is not fully in contact with the inner side of the first body 211 (except for the outlet 223), but the outlet 223 is in close contact with the inner side of the first body 211, so the second side of the first space 211s, which is formed when the first body 211 and the second body 221 are combined, can be sealed. Further, the outlet 223 is formed to sharply protrude at the end, so it can be effectively locked to the first locking portion 215 on the inner side of the first body 211.

The breakable membrane 224 is a film closing the outlet 223. Accordingly, the second side of the second space 221s is sealed by the piston 230 and the first side is sealed by the breakable membrane 224, so leakage of the second skincare agent can be prevented. Further, since the first side of the second space 221s is sealed by the breakable membrane 224, the second skincare agent in the second space 221s and the first skincare agent in the first space 211s can be stably kept therein without mixing. Further, (though will be described in detail bellow), if necessary, the breakable membrane can be rupture and opened, so the second skincare agent can be discharged out of the second space 221s and consequently discharged out of the outlet 213 through the first space 211s.

The second locking portion 225, similar to the first locking portion 215, is formed in a ring shape protruding from the inner side of the second body 221, and if a uses doesn't want, the second locking portion 225 fixes the position of the piston 230 so that the piston 230 is not inserted further than a predetermined position and the second skincare agent is not discharged. Similar to the first locking portion 215, the second locking portion 225 may be formed in one ring shape, or as show in FIGS. 4 and 6, it may be formed in a pair of ring shapes protruding from the inner side of the second body 221 and positioned close to each other.

Further, the second locking portion 225 may be formed at two or more positions on the second body 221. FIGS. 4 and 6 show an example in which lower and upper second locking portions 225a and 225 are formed at lower and upper positions. According to this configuration, it is possible to more stably fix the piston 230 at the predetermined position.

Finally, the piston cylinder 230 is described. As shown in FIG. 5, the piston cylinder 230 has a piston body 231, a piston flange 232, a breaking portion 233, and a piston locking portion 235.

The piston body 231 is inserted in the second body 221 and functions as the piston of a common syringe. The piston body 231 may be a solid member without a space therein. However, since the second skincare agent is kept in the empty space formed by combining the second cylinder 220 and the piston 230, an additional space may be formed at a first end portion of the piston body 231 to keep much second skincare agent. To this end, the piston body 23, as shown in FIGS. 4 and 7, has a space 232s connecting a first side and a second side of the piston body 231 and a closing wall 234 formed like a separation wall to close the space 231s may be formed in the space 232s at a position closer to the first side of the piston body 231. By forming the space 231s and the closing wall 234, as described above, it is possible to obtain an additive effect of reducing the weight of the piston body 231.

The piston flange 222 is formed at a second end of the piston body 231, and similar to the first flange 212 and the second flange 222, functions as a support that supports the hand of a user pressing the piston 230. Further, the piston flange 232 can also limit the insertion depth of the piston 230 by being locked to the second flange 222 when the piston 230 is inserted at a predetermined depth in the second cylinder 220.

The breaking portion 223 is formed at a first end of the piston body 231 to correspond to the breakable membrane 224 and is pointed at the end to be able to effectively rupture the breakable membrane 224. As described above, the first side of the second space 221s is sealed by the breakable membrane 224 and the second side is sealed by the piston 230 in the second cylinder 220. When the piston 230 is pushed over a predetermined depth in the second cylinder 220, the breaking portion 233 penetrates and ruptures the breakable membrane 224, so the first side of the second space 221s is opened. Accordingly, the second skincare agent kept in the second space 221s can flow into the first space 211s, and consequently, it can be discharged through the outlet 213.

The piston locking portion 235 corresponds to the second locking portion 225 and functions similar to the end of the outlet 233 having a diameter larger than that of the second body 221. That is, the piston locking portion 235 is formed in a ring shape protruding from the outer side of the piston body 231, and if user doesn't want, it fixes the position of the piston 230 to prevent the piston 230 from being inserted over a predetermined depth and unnecessarily rupturing the breakable membrane 224.

Further, as described above, the piston locking portion 235 corresponds to the second locking portion 225 and the second locking portion 225 may be formed at two or more positions, so the piston locking portion 235 may also be formed at two or more positions on the piston body 231. FIGS. 4 and 7 show an example in which lower and upper piston locking portions 235a and 235 are formed at lower and upper positions. According to this configuration, it is possible to more stably fix the piston 230 at the predetermined position.

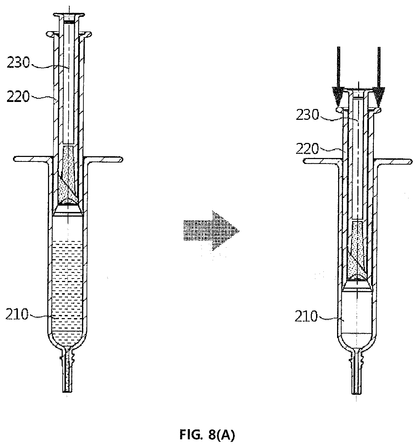

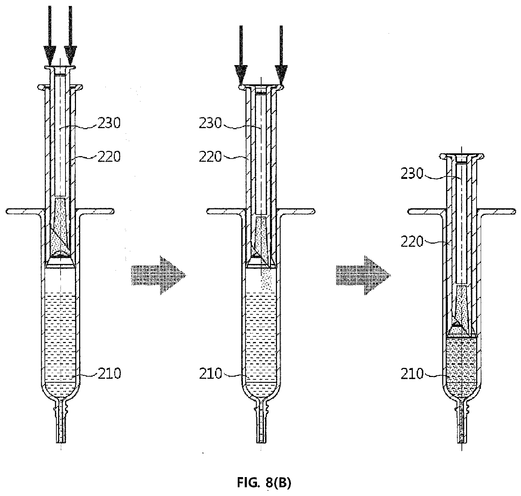

FIG. 8 shows embodiments of injecting a skincare agent, using the injector.

FIG. 8A shows an example of operating the injector when using only the first skincare agent without using the second skincare agent. As shown in the figure, when a user presses only the flange of the second cylinder 220 without touching the flange of the piston 230 (in the left one of FIG. 8A), only the first skincare agent in the first cylinder 210 is discharged through the outlet without the second skincare agent in the second cylinder 220 pressurized (in the right one in FIG. 8A).

FIG. 8B shows an example of operating the injector when using both of the first skincare agent and the second skincare agent. As shown in the figure, in this case, when a user presses the flange of the piston 220 (in the left one of FIG. 8B), the breakable membrane of the second cylinder is ruptured by the breaking portion of the piston and the second skincare agent in the second cylinder 220 is discharged into the first cylinder 210 (in the middle one of FIG. 8B), so the first skincare agent and the second skincare agent are mixed with each other. In this state, when the flange of the second cylinder 220 is further pressed, the mixture of the first skincare agent and the second skincare agent in the first cylinder 210 is discharged through the outlet (in the right one of FIG. 8B).

Further, an additional configuration for more easily keeping the parts of the facial mask kit is described.

The pouch 100 may further have an injector space 120 separated from the sheet space 110, as shown in FIG. 1. Further, the pouch 110, similar to the pouches of common facial masks, may be formed by stacking a pair of packaging materials and then thermally bonding the edges to form the sheet space 110. In this case, it is possible to form the injector space 120 separated from the sheet space 110 by further thermally bonding the centers of the pair of packaging materials with the edges thermally bonded. Accordingly, the injector 200 can be stably kept in the facial mask kit 100.

A pair of notched opening portions 140 may be formed at the left and right ends of the upper portion of the pouch 100, so a user can easily open the pouch by holding the portion over the opening portions and the tearing the pouch. Further, a perforated crease may be formed at the bonded portion 130 between the sheet space 110 and the injector space 120 so that the sheet space 110 and the injector space 120 can be easily separated when the injector 200 is used. According to this configuration, it is possible to prevent the sheet space 110 is unnecessarily opened when the pouch is opened to use the injector 220.

Further, the pouch 100 can be made of a common opaque material. In this case, the packaging material for the sheet space 110 may be opaque, but the packaging material for the injector space 120 may be transparent at least on one side. According to this configuration, it is a possible to visually check whether a skincare agent has leaked from the injector 200.

The present invention is not limited to the embodiments described above, may be used for various fields, and may be modified in various ways by those skilled in the art without departing from the spirit of the present invention described in claims.

### Industrial Applicability

According to the present invention, manufacturers also can change the contents of a product by changing the components to be kept in the injector, so no separate manufacturing line for each component is required and the cost for facilities can be remarkably reduced.

## Claims

1. A facial mask kit comprising:
a pouch having a sheet space for keeping a sheet therein;
an injector including a cylinder having an outlet at a first side and keeping a first skincare agent therein and a piston inserted in the cylinder from a second side of the cylinder;
an injection port disposed on a first side of the pouch to connect inside and outside of the sheet space,
wherein the skincare agent is injected into the sheet space through the injection port by the injector to uniformly soak the sheet.

2. The facial mask kit of claim 1, wherein the injector includes a first cylinder, a second cylinder inserted in the first cylinder, and a piston inserted in the second cylinder,
a first skincare agent is kept in a space between the first cylinder and the second cylinder, and
a second skincare agent is kept in a space between the second cylinder and the piston.

3. The facial mask kit of claim 2, wherein the first cylinder has: a first body having a first space therein and having a first side and a second side communicating with each other; an outlet formed at a first end of the first body; a first flange formed at a second end of the first body; and a first locking portion protruding in a ring shape from an inner side of the first body.

4. The facial mask kit of claim 3, wherein the first locking portion is formed in a pair of ring shapes protruding from the inner side of the first body and positioned close to each other.

5. The facial mask kit of claim 3, wherein the second cylinder has: a second body having a second space therein, having a first side and a second side communicating each other, and inserted in the first body; an outlet formed at a first end of the second body and having a larger diameter at an end than a diameter of the second body; a second flange formed at a second end of the second body; a breakable membrane formed as a separation membrane closing the outlet; and at least one second locking portion protruding in a ring shape from an inner side of the second body.

6. The facial mask kit of claim 5, wherein the second locking portion is formed in a pair of ring shapes protruding from the inner side of the second body and positioned close to each other.

7. The facial mask kit of claim 5, wherein the piston has: a piston body inserted in the second body; a breaking portion formed at a first end of the piston body and pointed at an end thereof; a piston flange formed at a second end of the piston body; and at least one piston locking portion protruding in a ring shape from an outer side of the piston body.

8. The facial mask kit of claim 7, wherein the piston body has a space connecting a first side and second side of the piston body and has a closing wall formed as a separation wall to close the space at a position closer to the first side of the piston body.

9. The facial mask kit of claim 1, wherein the injection port has: an injection port body having an empty space therein and having a first side and a second side communicating with other; an injection port flange formed at a first end of the injection port body and attached and fixed to the first side of the pouch; a muzzle formed at a second end of the injection port body; and a cap disposed over the muzzle to be opened and closed.

10. The facial mask kit of claim 9, wherein the injection port body is inclined with respect to a surface of the pouch.

11. The facial mask kit of claim 10, wherein the sheet is positioned closer to a bottom in the sheet space and the injection port is positioned closer to a top in the sheet space.

12. The facial mask kit of claim 11, wherein the injection port body is declined downward.

13. The facial mask kit of claim 1, wherein the pouch further has an injector space separated from the sheet space.

14. The facial mask kit of claim 13, wherein the pouch is formed by stacking a pair of packaging materials and then thermally bonding edges to form the sheet space.

15. The facial mask kit of claim 14, wherein the injector space separated from the sheet space of the pouch is formed by further thermally bonding centers of the pair of packaging materials with the edges thermally bonded.

16. The facial mask kit of claim 15, wherein a perforated crease is formed at the bonded portion between the sheet space and the injector space.

17. The facial mask kit of claim 14, wherein a pair of notched opening portions are formed at left and right ends of an upper portion of the pouch.

18. The facial mask kit of claim 13, wherein in the pouch, a packaging material for the sheet space is opaque, and
a packaging material for the injector space is transparent at least on one side.

19. The facial mask kit of claim 1, wherein the injector has an injector cap sealing the outlet.

20. The facial mask kit of claim 19, wherein the injector cap is thread-fastened to the outlet.

21. The facial mask kit of claim 20, wherein the injector cap has wings to be turned when being thread-fastened to the outlet.
